# EUROPEAN PATENT SPECIFICATION

(11) **EP 1 762 837 B1**
(45) Date of publication and mention of the grant of the patent: **06.01.2016**
(21) Application number: 06018956.0
(22) Date of filing: 11.09.2006
(51) Int. Cl.: G01N 7/00, G01N 33/00, G01N 7/14, G01N 33/14

(54) **Method and measuring device for determining the CO2 concentration in a CO2-containing liquid**
Verfahren und Vorrichtung zur Konzentrationsmessung einer Kohlendioxidhaltige Flüssigkeit
Procédé et dispositif pour mesurer la concentration du dioxyde de carbone dans un liquide

(30) Priority: 12.09.2005 NL 1029934
(43) Date of publication of application: 14.03.2007
(73) Proprietor: Haffmans B.V., 5928 PW Venlo (NL)
(72) Inventor: Martynowicz, Emile Thomas Martinus Johannes, 5925 HJ Venlo (NL); Geelen, Eric Henricus Christiaan, 5921 JS Venlo (NL); Günther, Christian Wilhelm, 5911 GT Venlo (NL); Holterman, Menno Martijn, 5911 BG Venlo (NL)
(74) Representative: Algemeen Octrooi- en Merkenbureau B.V.

(56) References cited:
- DE-A1- 4 322 017
- US-A- 4 179 918
- US-A- 5 220 513
- US-A- 5 426 593
- US-B2- 6 874 351

## Description

The invention relates to a method for determining the CO₂ concentration in a CO₂-containing liquid, in particular CO₂-containing (soft) drinks according to the preamble of claim 1.

The invention also relates to a measuring device for determining the CO₂ concentration in a CO₂-containing liquid, in particular CO₂-containing (soft) drinks according to the preamble of claim 10.

It is highly desirable that the correct CO₂ content of CO₂-containing beverages (beer or soft drinks) be known, because CO₂ content is an important quality parameter of the product, which is a determining factor for the taste, the foam quality and/or the preservative action, for example. It is essential, therefore, that an accurate CO₂ measurement be made in the case of beer and (soft) drink processes. A usual method in that connection is carrying out the CO₂ measurement on the basis of the temperature and the partial pressure of CO₂, based on Henry's law.

An example of such measurement based on Henry's law is disclosed in US-A-4179918. In this prior art document a method and an apparatus for measuring the carbon dioxide content in beer is disclosed, in which a beer sample is separated in a vessel. Subsequently the pressure of the carbon dioxide atmosphere being created above said beer sample in the vessel is measured.

In beer, for example, the equilibrium pressure above the liquid is almost fully determined by the CO₂ gas, so that the CO₂ partial pressure substantially corresponds to the total pressure above the liquid. Thus, the CO₂ concentration can be determined from the total pressure above the liquid (after an equilibrium has been attained between the liquid and the gas) and the temperature.

Because the beer brewing process is a substantially air-free process and no other gases besides carbon dioxide are present, a simple but accurate pressure and temperature measurement suffices for determining the CO₂ content in the beer. These operating conditions are in particular based on the fact that beer must be filled with carbon dioxide without air as much as possible, so that there will be hardly any inclusion of other gases, such as nitrogen and oxygen.

In the production of carbonated (soft) drinks, however, to which CO₂ is added, a simple pressure and temperature measurement as described above frequently does not suffice, because larger amounts of air may be present in the production process or be included, for example upon filling, when such production processes are used.

In such an environment, a simple pressure and temperature measurement may lead to considerable measuring errors, therefore.

The object of the method is to provide a measuring technique that is especially intended for use in preparation process for carbonated (soft) drinks, which measuring technique does not exhibit the above-described drawbacks.

According to the invention, the method is characterized by the steps of
a) providing a predetermined amount of CO₂-containing liquid with a predetermined volume of a head gas that is in gas equilibrium with the liquid;
b) determining the total pressure of the head gas;
c) determining the partial pressure of oxygen (O₂) of the head gas;
d) determining the partial pressure of nitrogen (N₂) of the head gas;
e) deriving the partial pressure of carbon dioxide (CO₂) of the head gas from the total pressure and the partial pressures of oxygen and nitrogen; and
f) deriving the CO₂ concentration in the CO₂-containing liquid from the derived partial pressure of carbon dioxide wherein step c) comprises the steps of
   c1) measuring the oxygen content in the CO2-containing liquid;
   c2) measuring the temperature; and
   c3) deriving the partial pressure of oxygen in the head gas on the basis of the information obtained in steps c1) and c2).

In this way the CO₂ concentration in the liquid can be determined in an effective but above all accurate manner whilst avoiding any errors in the measurement.

In a special embodiment of the method according to the invention, step d) comprises the deriving of the partial pressure of nitrogen from the partial pressure of oxygen.

For the sake of measuring accuracy, the amount of CO₂-containing liquid and the head gas need to be in liquid/gas equilibrium with each other. In this way a direct relation is obtained between the CO₂ concentration in the head gas that is to be determined and the CO₂ concentration in the liquid.

In order to obtain this equilibrium, step a) may according to the invention comprise the shaking or stirring of said amount of CO₂-containing liquid for a specified period of time or the electrolysis of said amount of CO₂-containing liquid for a specified period of time. Another functional method is one wherein said amount of CO₂-containing liquid is exposed to ultrasound for a specified period of time or wherein a vacuum space is created above said amount of CO₂-containing liquid.

In a special embodiment, step f) may comprise the steps of
f1) measuring the temperature; and
f2) deriving the CO₂ concentration in the CO₂-containing liquid on the basis of the information obtained in steps e) and f1).

According to the invention, the measuring device for determining the CO₂ concentration in a CO₂-containing liquid, in particular CO₂-containing (soft) drinks, comprises a housing provided with at least
a sampling opening, which can be placed into communication with a mixture composed of a specified amount of the CO₂-containing liquid as well as a specified amount of head gas being in equilibrium with the liquid;
a pressure sensor for measuring the total pressure of the head gas;
determining means arranged for determining the partial pressure of oxygen (O₂) and the partial pressure of nitrogen (N₂); and
deriving means arranged for deriving the partial pressure of carbon dioxide (CO₂) from the total pressure and the partial pressures of oxygen and nitrogen.

These technical aspects make it possible to determine the CO₂ concentration in the liquid to be sampled in an effective but above all accurate manner whilst avoiding any measuring errors. The latter is important in particular in the preparation of carbon dioxide-containing (soft) drinks, when other gases, such as air (containing oxygen and nitrogen), are present in the process besides other gases.

In a specific embodiment, the determining means comprise a concentration sensor for measuring the oxygen concentration in the specified amount of the CO₂-containing liquid, or a temperature sensor for measuring the temperature of the specified amount of the CO₂-containing liquid.

More specifically, the determining means are arranged for deriving the partial pressure of oxygen in the head gas from the oxygen concentration and the temperature as measured, said determining means in particular being arranged for deriving the partial pressure of nitrogen from the partial pressure of oxygen.

A functional embodiment is obtained if the deriving means are according to the invention arranged for deriving the CO₂ concentration in the CO₂-containing liquid on the basis of the derived partial pressure of carbon dioxide and the measured temperature.

In another functional embodiment, the housing furthermore comprises a sampling space for containing a specified amount of the head gas, wherein the pressure sensor is disposed within the sampling space so as to achieve an effective determination of the CO₂ concentration in the liquid.

In addition to that, the sampling opening may comprise a coupling for connecting a liquid pipe thereto.

The invention will now be explained in more detail with reference to a drawing, in which:
Figure 1 shows a first embodiment of a device for measuring the CO₂ concentration in a CO₂-containing liquid by means of the method according to the invention;
Figure 2 shows a second embodiment of a device for measuring the CO₂ concentration in a CO₂-containing liquid by means of the method according to the invention.

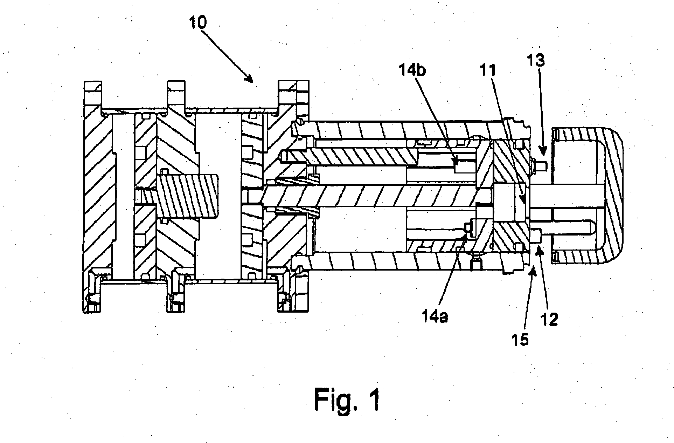

In figure 1 a first embodiment is shown of a measuring device for measuring the CO₂ concentration in a CO₂-containing liquid by means of the method according to the invention. The measuring device 10 comprises a housing as well as a sampling space 15, which can be placed into communication with a mixture composed of a specified amount of the CO₂-containing liquid as well as a predetermined volume of gas space or sampling space (the gas is also referred to as "head gas"), which is in liquid/gas equilibrium with the liquid.

Once the sampling space has been closed and the head gas space above the liquid has been created by "generating a vacuum", the total pressure of the head gas can be determined by a pressure sensor 11. The housing is furthermore provided with determining means 12-13-14a at least consisting of a concentration sensor 12, which determines the oxygen concentration of said specified amount of the CO₂-containing liquid, and a temperature sensor 13 for determining the current temperature of the head gas and/or said specified amount of the CO₂-containing liquid.

The concentration sensor 12 may be based on various measuring techniques that are known per se, for example an optical measuring principle.

The determining means 14a are furthermore provided with suitable electronics arranged for determining the partial pressure of oxygen in the head gas on the basis of the measured oxygen concentration and the current temperature. The electronics of the determining means 14a is also arranged for deriving the partial pressure of nitrogen (N₂) in the head gas on the basis of the determined partial pressure of oxygen (O₂). It is assumed in this connection that the proportion between the partial pressure of oxygen and the partial pressure of nitrogen is known in advance, amounting to 20 : 80, for example.

Furthermore, the measuring device 10 according to the invention comprises deriving means 14b in the form of electronics, which are arranged for deriving the partial pressure of carbon dioxide of the head gas from the total pressure (as measured by the pressure sensor 11) and the partial pressures of oxygen and nitrogen (as determined by the determining means 14a).

By determining the partial pressures of oxygen and nitrogen in addition to the total pressure of the head gas, a more accurate determination of the partial pressure of carbon dioxide of the head gas can be realised, so that the measuring device 10 will be very suitable for use in preparation processes of carbonated (soft) drinks, when large amounts of air are already present in the process, for example when the beverage is being filled into the bottles.

This makes it possible to obtain an accurate measurement of the CO₂ concentration in the carbonated drink, the more so because a correction can be made for the amount of air (mainly consisting of oxygen and nitrogen) that is present, so that the measurement obtained will not be incorrect.

On the basis of the derived partial pressure of carbon dioxide, the determining means 14b can determine the CO₂ concentration in the carbon dioxide-containing liquid with the aid of the temperature sensor 13 and in particular the measured temperature.

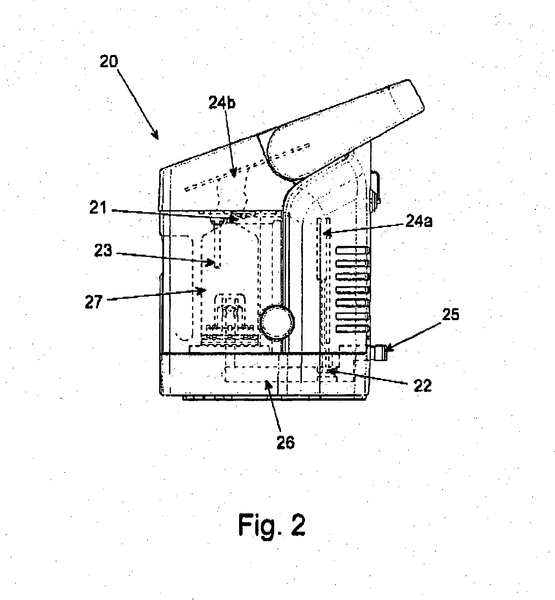

In figure 2 a second embodiment is shown, in which the measuring device 20 also comprises a housing, which is provided with a sampling space 27 in this case, which is in communication with the sampling space 25 via a channel 26. In this embodiment, the sampling opening 25 is in the form of a coupling, which enables a simple connection to a pipe through which the liquid to be sampled flows.

A pressure sensor 21 is provided in the sampling space 27, which sensor determines the total pressure in the sampling space 27. Said sampling space is in equilibrium with an added head gas space. The head gas is led to the sampling space 27 via the coupling 25 and the pipe 26 in that case and is in equilibrium with the carbon dioxide-containing liquid that is present in the pipe 26 and the coupling 25. A concentration sensor 22 is mounted in the pipe 26 for determining the concentration of oxygen in the liquid. The concentration sensor 12 may be based on various measuring techniques that are known per se, such as an optical measuring principle.

Also present in the sampling space 27 is a temperature sensor 23 for determining the current temperature of the head gas that is present in the head gas space.

Analogously to the embodiment that is shown in figure 1, the measuring device 20 is provided with determining means 24a for determining the partial pressure of oxygen in the head gas space on the basis of the measured oxygen concentration and the current temperature. Likewise, the deriving means 24b are arranged for deriving the partial pressure of nitrogen (N₂) in the gas space on the basis of the determined partial pressure of oxygen (O₂). In this case, too, it is assumed that the proportion between the partial pressure of oxygen and the partial pressure of nitrogen is a known proportion.

The partial pressure of oxygen measured by the determining means 24a and the derived partial pressure of nitrogen and the total pressure as measured (by the pressure sensor 21) are used by the deriving means 24b that are also present in the measuring device 20 for deriving the partial pressure of carbon dioxide of the head gas that is present in the sampling space 27. The CO₂ concentration in the CO₂-containing liquid can then be determined fairly accurately on the basis of the current temperature as also measured by the temperature sensor 23.

With the method and the device according to the invention it is assumed that an equilibrium exists between the liquid to be sampled and the head gas. This makes it necessary to effect an equilibrium between the liquid to be sampled and the gas space (head gas) present above the liquid by shaking or stirring or by electrolysis prior to the measurement, so that there will be a direct relation between the CO₂ concentration in the gas space (head gas) and the CO₂ concentration in the liquid to be sampled. Another functional method is a method wherein the amount of CO₂-containing liquid in the sampling space is exposed to ultrasound for a specified period of time or wherein a vacuum space is created around the amount of CO₂-containing liquid.

When the latter method is used, as shown in figure 1, the sampling space is enlarged, as a result of which a vacuum space which realises the liquid/gas equilibrium between the liquid and the head gas is created in the sampling space. To that end a part of the wall of the sampling space may be movable, for example by configuring it as the piston of a piston/a cylinder combination. The sampling space will be formed by the cylinder chamber in that case, which can be enlarged (or reduced) by moving the piston. In the case of an enlargement, a "vacuum" is created, thereby realising the aforesaid liquid/gas equilibrium between the liquid and the head gas.

When carrying out the measurement according to the invention with a bottle in which the carbon dioxide-containing liquid is already contained, the amount of head gas or the gas space is also present above the liquid (and in the bottle) already. When carrying out measurements in process piping of a processing arrangement for producing such carbon dioxide-containing (soft) drinks, it suffices to draw a specified amount of carbon dioxide-containing liquid from the pipe (via the sampling openings 15 or 25 in figures 1 and 2).

In this latter measuring situation, said sampling space needs to be created, for example by adding a vacuum space or an expansion space. In one embodiment, the sampling space may have an adjustable volume, for example in that the sampling space comprises at least one movable wall part, which can be moved in a suitable manner, for example by means of a piston rod, so that a sample of the head gas can be taken in this manner.

## Claims

1. A method for determining the CO₂ concentration in a CO₂-containing liquid, in particular CO₂-containing (soft) drinks, comprising the steps of
a) providing a predetermined amount of CO₂-containing liquid with a predetermined volume of a head gas that is in gas equilibrium with the liquid, wherein the head gas consists of carbon dioxide and air, with the air mainly consisting of oxygen and nitrogen;
b) measuring the total pressure of the head gas;
c) determining the partial pressure of oxygen (O₂) of the head gas;
d) determining the partial pressure of nitrogen (N₂) of the head gas;
e) deriving the partial pressure of carbon dioxide (CO₂) of the head gas from the total pressure and the partial pressures of oxygen and nitrogen; and
f) deriving the CO₂ concentration in the CO₂-containing liquid from the derived partial pressure of carbon dioxide, wherein step c) comprises the steps of
c1) measuring the oxygen content in the CO₂-containing liquid;
c2) measuring the temperature; and
c3) deriving the partial pressure of oxygen in the head gas on the basis of the information obtained in steps c1) and c2).

2. A method according to claim 1, **characterised in that** step d) comprises the step of deriving of the partial pressure of nitrogen from the partial pressure of oxygen.

3. A method according to claim 1 or 2, **characterised in that** step a) comprises the step of shaking or stirring of said amount of CO₂-containing liquid and the head gas for a specified period of time.

4. A method according to claim 1 or 2, **characterised in that** step a) comprises the step of electrolysis of said amount of CO₂-containing liquid for a specified period of time.

5. A method according to claim 1 or 2, **characterised in that** step a) comprises the exposure of said amount of CO₂-containing liquid to ultrasound for a specified period of time.

6. A method according to claim 1 or 2, **characterised in that** step a) comprises the creation of a vacuum space above said amount of CO₂-containing liquid.

7. A method according to claim 1 or 2, **characterised in that** step a) comprises the addition of an expansion space to the sampling space.

8. A method according to any one or more of the preceding claims, **characterised in that** steps d) and/or e) and/or f) comprise(s) the step of correcting the CO₂ concentration for other gases and/or substances that can be derived from the partial pressure O₂.

9. A method according to any one or more of the preceding claims, **characterised in that** step f) comprises the steps of
f1) measuring the temperature; and
f2) deriving the CO₂ concentration in the CO₂-containing liquid on the basis of the information obtained in steps e) and f1).

10. A measuring device for determining the CO₂ concentration in a CO₂-containing liquid, in particular CO₂-containing (soft) drinks, comprising a housing provided with at least
a sampling opening, which can be placed into communication with a mixture composed of a specified amount of the CO₂-containing liquid as well as a specified amount of head gas being in equilibrium with the liquid, wherein the head gas consists of carbon dioxide and air, with the air mainly consisting of oxygen and nitrogen;
a pressure sensor for measuring the total pressure of the head gas; determining means arranged for determining the partial pressure of oxygen (O₂) and the partial pressure of nitrogen (N₂); and
deriving means arranged for deriving the partial pressure of carbon dioxide (CO₂) from the total pressure and the partial pressures of oxygen and nitrogen and for deriving the CO₂ concentration in the CO₂-containing liquid on the basis of the derived partial pressure of carbon dioxide and the measured temperature, wherein
the determining means comprise a concentration sensor for measuring the oxygen concentration in the specified amount of the CO₂-containing liquid, as well as
a temperature sensor for measuring the temperature of the specified amount of the CO₂-containing liquid, and wherein
the determining means are arranged for deriving the partial pressure of oxygen in the head gas from the oxygen concentration and the temperature as measured.

11. A measuring device according to claim 10, **characterised in that** the determining means are arranged for deriving the partial pressure of nitrogen from the partial pressure of oxygen.

12. A measuring device according to any one or more of the claims 10-11, **characterised in that** the housing furthermore comprises a sampling space for containing at least a specified amount of the head gas

13. A measuring device according to claim 12, **characterised in that** the pressure sensor is disposed within the sampling space.

14. A measuring device according to any one or more of the claims 10-13, **characterised in that** the sampling opening comprises a coupling for connecting a liquid pipe thereto.

15. A measuring device according to any one or more of the claims 10-14, **characterised in that** the sampling space has an adjustable volume.

16. A measuring device according to claim 15, **characterised in that** the sampling space has at least one movable wall part.

17. A measuring device according to any one or more of the claims 10-16, **characterised in that** the device comprises an expansion space that can be placed in communication with the sampling space.

18. A measuring device according to any one or more of the claims 10-17, **characterised in that** said deriving means are arranged for correcting the CO₂ concentration for other gases and/or substances.

## Patentansprüche

1. Verfahren zum Bestimmen der CO₂-Konzentration einer CO₂-haltigen Flüssigkeit, insbesondere eines CO₂-haltigen (alkoholfreien) Getränks, das die Stufen:
a) Bereitstellen einer vorher festgelegten Menge einer CO₂-haltigen Flüssigkeit mit einem vorher festgelegten Volumen eines Kopfraumgases, das sich in einem Gas-Gleichgewicht mit der Flüssigkeit befindet, wobei das Kopfraumgas aus Kohlendioxid und Luft und die Luft im Wesentlichen aus Sauerstoff und Stickstoff besteht,
b) Messen des Gesamtdrucks des Kopfraumgases,
c) Bestimmen des Partialdrucks des Sauerstoffs (O₂) des Kopfraumgases,
d) Bestimmen des Partialdrucks des Stickstoffs (N₂) des Kopfraumgases,
e) Ableiten des Partialdrucks des Kohlendioxids (CO₂) des Kopfraumgases aus dem Gesamtdruck und den Partialdrücken von Sauerstoff und Stickstoff und
f) Ableiten der CO₂-Konzentration der CO₂-haltigen Flüssigkeit aus dem abgeleiteten Kohlendioxidpartialdruck umfasst, wobei Stufe c) die Stufen:
c1) Messen des Sauerstoffgehalts der CO₂-haltigen Flüssigkeit,
c2) Messen der Temperatur und
c3) Ableiten des Sauerstoffpartialdrucks des Kopfraumgases auf der Grundlage der in den Stufen c1) und c2) erhaltenen Informationen

2. Verfahren nach Anspruch 1, **dadurch gekennzeichnet, dass** Stufe d) die Stufe des Ableitens des Stickstoffpartialdrucks aus dem Sauerstoffpartialdrucks umfasst.

3. Verfahren nach Anspruch 1 oder 2, **dadurch gekennzeichnet, dass** Stufe a) die Stufe des Schüttelns oder Rührens der Menge an CO₂-haltiger Flüssigkeit und Kopfraumgas einen festgelegten Zeitraum lang umfasst.

4. Verfahren nach Anspruch 1 oder 2, **dadurch gekennzeichnet, dass** Stufe a) die Stufe der Elektrolyse der Menge an CO₂-haltiger Flüssigkeit einen festgelegten Zeitraum lang umfasst.

5. Verfahren nach Anspruch 1 oder 2, **dadurch gekennzeichnet, dass** Stufe a) das Beschallen der Menge an CO₂-haltiger Flüssigkeit mit Ultraschall einen festgelegten Zeitraum lang umfasst.

6. Verfahren nach Anspruch 1 oder 2, **dadurch gekennzeichnet, dass** Stufe a) das Erzeugen eines Vakuumraumes über der Menge an CO₂-haltiger Flüssigkeit umfasst.

7. Verfahren nach Anspruch 1 oder 2, **dadurch gekennzeichnet, dass** Stufe a) das Hinzufügen eines Ausdehnungsraumes zu dem Probenraum umfasst.

8. Verfahren nach einem oder mehreren der vorhergehenden Ansprüche, **dadurch gekennzeichnet, dass** Stufe d) und/oder e) und/oder f) die Stufe der Korrektur der CO₂-Konzentration hinsichtlich anderen Gasen und/oder Substanzen, die sich aus dem O₂-Partialdruck ableiten lassen, umfasst/umfassen.

9. Verfahren nach einem oder mehreren der vorhergehenden Ansprüche, **dadurch gekennzeichnet, dass** Stufe f) die Stufen:
f1) Messen der Temperatur und
f2) Ableiten der CO0₂-Konzentration der CO₂-haltigen Flüssigkeit auf der Grundlage der in den Stufen e) und f1) erhaltenen Informationen
umfasst.

10. Messvorrichtung zum Bestimmen der CO₂-Konzentration einer CO₂-haltigen Flüssigkeit, insbesondere eines CO₂-haltigen (alkoholfreien) Getränks, welche ein Gehäuse umfasst, das mit mindestens:
- einer Probenöffnung, die in Verbindung mit einem Gemisch gebracht werden kann, das sich aus einer festgelegten Menge der CO₂-haltigen Flüssigkeit sowie einer festgelegten Menge an Kopfraumgas, das sich im Gleichgewicht mit der Flüssigkeit befindet, zusammensetzt, wobei das Kopfraumgas aus Kohlendioxid und Luft und die Luft im Wesentlichen aus Sauerstoff und Stickstoff besteht,
- einem Drucksensor zum Messen des Gesamtdrucks des Kopfraumgases,
- Bestimmungsmitteln, die für das Bestimmen des Partialdrucks von Sauerstoff (O₂) und Stickstoff (N₂) eingerichtet sind, und
- Ableitungsmitteln, die für das Ableiten des Partialdrucks des Kohlendioxids (CO₂) aus dem Gesamtdruck und dem Sauerstoff- und dem Stickstoffpartialdruck und für das Ableiten der CO₂-Konzentration der CO₂-haltigen Flüssigkeit auf der Grundlage von dem abgeleiteten Kohlendioxidpartialdruck und der gemessenen Temperatur eingerichtet sind, versehen ist, wobei
- die Bestimmungsmittel einen Konzentrationssensor für die Messung der Sauerstoffkonzentration in der festgelegten Menge der CO₂-haltigen Flüssigkeit sowie
- einen Temperatursensor für die Messung der Temperatur der festgelegten Menge der CO₂-haltigen Flüssigkeit umfassen und wobei
die Bestimmungsmittel zum Ableiten des Sauerstoffpartialdrucks des Kopfraumgases aus der gemessenen Sauerstoffkonzentration und Temperatur eingerichtet sind.

11. Messvorrichtung nach Anspruch 10, **dadurch gekennzeichnet, dass** die Bestimmungsmittel zum Ableiten des Stickstoffpartialdrucks aus dem Sauerstoffpartialdruck eingerichtet sind.

12. Messvorrichtung nach einem oder mehreren der Ansprüche 10 und 11, **dadurch gekennzeichnet, dass** das Gehäuse weiterhin einen Probenraum zum Aufnehmen mindestens einer festgelegten Menge an Kopfraumgas umfasst.

13. Messvorrichtung nach Anspruch 12, **dadurch gekennzeichnet, dass** der Drucksensor in dem Probenraum angeordnet ist.

14. Messvorrichtung nach einem oder mehreren der Ansprüche 10 bis 13, **dadurch gekennzeichnet, dass** die Probenöffnung einen Anschluss für das Anschließen einer Flüssigkeitsleitung an sie umfasst.

15. Messvorrichtung nach einem oder mehreren der Ansprüche 10 bis 14, **dadurch gekennzeichnet, dass** das Volumen des Probenraumes einstellbar ist.

16. Messvorrichtung nach Anspruch 15, **dadurch gekennzeichnet, dass** der Probenraum mindestens einen beweglichen Wandungsteil besitzt.

17. Messvorrichtung nach einem oder mehreren der Ansprüche 10 bis 16, **dadurch gekennzeichnet, dass** die Vorrichtung einen Ausdehnungsraum umfasst, der in Verbindung mit dem Probenraum gebracht werden kann.

18. Messvorrichtung nach einem oder mehreren der Ansprüche 10 bis 17, **dadurch gekennzeichnet, dass** die Ableitungsmittel zum Korrigieren der CO₂-Konzentration hinsichtlich anderer Gase und/oder Substanzen eingerichtet sind.

## Revendications

1. Procédé de détermination de la concentration en CO₂ dans un liquide contenant du CO₂, en particulier des boissons (non alcoolisées) contenant du CO₂, comprenant les étapes de :
a) fourniture à une quantité prédéterminé de liquide contenant du CO₂ d'un volume prédéterminé d'un gaz de tête qui est en équilibre gazeux avec le liquide, dans lequel le gaz de tête est constitué de dioxyde de carbone et d'air, l'air étant principalement constitué d'oxygène et d'azote ;
b) mesure de la pression totale du gaz de tête ;
c) détermination de la pression partielle d'oxygène (O₂) du gaz de tête ;
d) détermination de la pression partielle d'azote (N₂) du gaz de tête ;
e) dérivation de la pression partielle de dioxyde de carbone (CO₂) du gaz de tête de la pression totale et des pressions partielles d'oxygène et d'azote ; et
f) dérivation de la concentration en CO₂ dans le liquide contenant du CO₂ de la pression partielle dérivée du dioxyde de carbone, dans lequel l'étape c) comprend les étapes de :
c1) mesure de la teneur en oxygène dans le liquide contenant le CO₂ ;
c2) mesure de la température ; et
c3) dérivation de la pression partielle d'oxygène dans le gaz de tête sur la base des informations obtenues dans les étapes c1) et c2).

2. Procédé selon la revendication 1, **caractérisé en ce que** l'étape d) comprend l'étape de dérivation de la pression partielle d'azote de la pression partielle d'oxygène.

3. Procédé selon la revendication 1 ou 2, **caractérisé en ce que** l'étape a) comprend l'étape d'agitation ou de mélange de ladite quantité de liquide contenant du CO₂ et du gaz de tête pendant une durée spécifiée.

4. Procédé selon la revendication 1 ou 2, **caractérisé en ce que** l'étape a) comprend l'étape d'électrolyse de ladite quantité de liquide contenant du CO₂ pendant une durée spécifiée.

5. Procédé selon la revendication 1 ou 2, **caractérisé en ce que** l'étape a) comprend l'exposition de ladite quantité de liquide contenant du CO₂ à des ultrasons pendant une durée spécifiée.

6. Procédé selon la revendication 1 ou 2, **caractérisé en ce que** l'étape a) comprend la création d'un espace sous vide au-dessus de ladite quantité de liquide contenant du CO₂.

7. Procédé selon la revendication 1 ou 2, **caractérisé en ce que** l'étape a) comprend l'ajout d'un espace d'expansion à l'espace d'échantillonnage.

8. Procédé selon l'une quelconque ou plus des revendications précédentes, **caractérisé en ce que** les étapes d) et/ou e) et/ou f) comprennent l'étape de correction de la concentration en CO₂ pour d'autres gaz et/ou substances qui peut être dérivée de la pression partielle d'O₂.

9. Procédé selon l'une quelconque ou plus des revendications précédentes, **caractérisé en ce que** l'étape f) comprend en outre les étapes de :
f1) mesure de la température ; et
f2) dérivation de la concentration en CO₂ dans le liquide contenant du CO₂ sur la base des informations obtenues dans les étapes e) et f1).

10. Dispositif de mesure pour déterminer la concentration en CO₂ dans un liquide contenant du CO₂, en particulier des boissons (non alcoolisées) contenant du CO₂, comprenant un logement doté d'au moins
une ouverture d'échantillonnage, qui peut être placée en communication avec un mélange composé d'une quantité spécifiée de liquide contenant du CO₂ ainsi qu'une quantité spécifiée de gaz de tête en équilibre avec le liquide, dans lequel le gaz de tête est constitué de dioxyde de carbone et d'air, l'air étant principalement constitué d'oxygène et d'azote ;
un capteur de pression pour mesurer la pression totale du gaz de tête ;
un moyen de détermination agencé pour déterminer la pression partielle d'oxygène (O₂) et la pression partielle d'azote (N₂) ; et
un moyen de dérivation agencé pour dériver la pression partielle de dioxyde de carbone (CO₂) de la pression totale et des pressions partielles d'oxygène et d'azote et pour dériver la concentration en CO₂ dans le liquide contenant le CO₂ sur la base de la pression partielle dérivée de dioxyde de carbone et de la température mesurée, dans lequel
le moyen de détermination comprend un capteur de concentration pour mesurer la concentration en oxygène dans la quantité spécifiée de liquide contenant du CO₂, ainsi que
un capteur de température pour mesurer la température de la quantité spécifiée du liquide contenant le CO₂, et dans lequel
le moyen de détermination est disposé pour dériver la pression partielle d'oxygène dans le gaz de tête de la concentration en oxygène et de la température telle que mesurée.

11. Dispositif de mesure selon la revendication 10, **caractérisé en ce que** le moyen de détermination est agencé pour dériver la pression partielle d'azote de la pression partielle d'oxygène.

12. Dispositif de mesure selon l'une quelconque ou plus des revendications 10 à 11, **caractérisé en ce que** le logement comprend en outre un espace d'échantillonnage contenant au moins une quantité spécifiée de gaz de tête.

13. Dispositif de mesure selon la revendication 12, **caractérisé en ce que** le capteur de pression est disposé à l'intérieur de l'espace d'échantillonnage.

14. Dispositif de mesure selon l'une quelconque ou plus des revendications 10 à 13, **caractérisé en ce que** l'ouverture d'échantillonnage comprend un couplage pour raccorder un tuyau pour liquide à celui-ci.

15. Dispositif de mesure selon l'une quelconque ou plus des revendications 10 à 14, **caractérisé en ce que** l'espace d'échantillonnage a un volume ajustable.

16. Dispositif de mesure selon la revendication 15, **caractérisé en ce que** l'espace d'échantillonnage a au moins une paroi amovible.

17. Dispositif de mesure selon l'une quelconque ou plus des revendications 10 à 16, **caractérisé en ce que** le dispositif comprend un espace d'expansion qui peut être placé en communication avec l'espace d'échantillonnage.

18. Dispositif de mesure selon l'une quelconque ou plus des revendications 10 à 17, **caractérisé en ce que** ledit moyen de dérivation est agencé pour corriger la concentration en CO₂ pour d'autres gaz et/ou substances.
